# EUROPEAN PATENT SPECIFICATION

(11) **EP 0 925 356 B1**
(45) Date of publication and mention of the grant of the patent: **28.12.2005**
(21) Application number: 97924012.4
(22) Date of filing: 15.05.1997
(51) Int. Cl.: C12N 5/08, A61K 35/14, C07K 16/46

(54) **NEW ANTIGEN PRESENTING CELLS, A PROCESS FOR PREPARING THE SAME AND THEIR USE AS CELLULAR VACCINES**
ANTIGENPRÄSENTIERENDE ZELLEN, EIN VERFAHREN ZUR DEREN HERSTELLUNG UND DEREN VERWENDUNG ALS ZELLULÄRE IMPFSTOFFEN
NOUVELLES CELLULES PRESENTANT L'ANTIGENE, PROCEDE DE PREPARATION ASSOCIE, ET LEUR UTILISATION COMME VACCINS CELLULAIRES

(30) Priority: 21.05.1996 EP 96401099
(43) Date of publication of application: 30.06.1999
(73) Proprietor: I.D.M. IMMUNO-DESIGNED MOLECULES, 75011 Paris (FR)
(72) Inventor: CHOKRI, Mohamed, F-67000 Strasbourg (FR); BARTHOLEYNS, Jacques, F-91440 Bures-sur-Yvette (FR); ROMET-LEMONNE, Jean-Loup, F-75003 Paris (FR)
(74) Representative: Grosset-Fournier, Chantal Catherine
(86) International application number: PCT/EP1997/002703
(87) International publication number: WO 1997/044441

(56) References cited:
- WO-A-92/05793
- WO-A-94/26875
- WO-A-96/22781
- ANTICANCER RESEARCH, vol. 14, no. 6B, November 1994, ATHENS, GREECE, pages 2673-2676, XP000608444 J. BARTHOLEYNS ET AL.: "Immune control of neoplasia by adoptive transfer of macrophages: Potentiality for antigen presentation and gene transfer."
- IMMUNOLOGY, vol. 70, no. 2, June 1990, OXFORD, GB, pages 186-190, XP000608448 H. OKAMOTO ET AL.: "Possible involvement of adenosine 3':5'-cyclic monophosphate and extracellular calcium ions in histamine stimulation of interleukin-1 release from macrophage-like P388D1 cells."
- IMMUNOBIOLOGY, vol. 195, no. 4-5, October 1996, STUTTGART, GERMANY, pages 550-562, XP002039948 J. BARTOLEYNS ET AL.: "Immune therapy with macrophages: Present status and critical requirements for implementation."

## Description

The invention relates to new antigen presenting cells, a process for preparing the same and their use as cellular vaccines.

Macrophages are recognized since their description by Metchnikoff (Immunity in Infective Diseases, Cambridge Press, 1905) as cells which very effectively phagocytose (interiorise) and digest exogenous particles (antigens, cell debris, bacteria). They also secrete a variety of immunoeffector monokines. They have therefore a central role in initiating the non-specific and the specific immune responses. The recovery of large quantities of human macrophages differentiated in culture from blood monocytes has already been described (International application N° PCT/EP93/01232, refs. 1-6). These macrophages express typical antigens and functions and have been fully characterized (refs. 7-14).

Macrophages activated in the presence of IFNγ (Macrophage Activated Killers : MAK) elicited selective cytostasis and cytotoxicity for a large number of human tumors even at low effector/target ratio. In murine models, murine activated macrophages given locally in the tumor or its vicinity infiltrated the tumor mass, inhibited tumor growth and decreased metastatic development. Human macrophages also inhibited the growth of human tumors engrafted in nude or SCID mice ; this effect was achieved after local or systemic injection of a low number of macrophages (less than 1 million MAK) for mice with macroscopic tumors (ref. 15-20).

Patients with metastatic cancer were infused systemically or intraperitoneally with 10⁸ to 4x10⁹ autologous MAK. Tumoricidal monocytes (AKM) were also infused intraperitoneally in patients with colorectal carcinomas. The clinical tolerance of MAK was excellent with minor side effects such as low grade fever and chills and no autoimmune nor acute phase reactivity. No complete antitumoral response was reported ; improved prognosis and prolonged disease free intervals were described after intraperitoneal injection of AKM, while tumor necrosis, stabilisation, reduction of ascitic fluid and change in chemioresistance were seen after MAK therapy (refs. 21 - 26).

The recognized limitation of these macrophages is that they are not very potent in the priming of a specific immune response against a specific exogenous antigen or tumor by stimulation of MHC class I restricted cytotoxic T lymphocytes (CD8 +). They are more efficient in presenting antigens in the context of MHC class II molecules to T helper lymphocytes (CD4 +).

However these macrophages have the potential to process and present soluble antigens by the exogenous pathway of phagocytes but high concentrations of proteins or antigens are required (refs. 38 - 42, 45 - 47, 50).

Cells expressing these functions of phagocytosis, digestion, processing, and presentation at a high level would be required for the development of cellular vaccines.

Dendritic cells are characterized by their morphology (dendrites) and a few membrane antigens, and are considered as professional antigen - presenting cells resident in tissues. They are the most potent cells for the stimulation of primary T - lymphocyte immune responses (refs. 43 - 44, 47 - 49, + Dendritic cells in fundamental and clinical immunology. 1995, Plenum Press N.Y., Banchereau and Schmitt Editors).

Dendritic cell precursors arise from bone marrow and can be found in blood and lymph. Dendritic cells derived from these origins can be obtained by culture in the presence of GM - CSF + IL4 + TNF. They will then exhibit differences related to their maturation state and microenvironment.

The dendritic cells which can be derived from blood are most potent to induce allogenic mixed lymphocyte reactions and to stimulate naive T lymphocytes. However, these classical dendritic cells are technically relatively difficult to obtain and are poorly phagocytozing.

In contrast to macrophages, they do not express of CD14 and CD64 (high affinity Fγ receptor).

To circumvent the problem of poor phagocytoses and processing of particular antigens by dendritic cells, these cells have been pulsed with small peptides fixed on MHC-I molecules to induce primary immune reaction and vaccination against new antigenic peptides (ref. 51).

Obtaining dendritic cells by *in vitro* differentiation requires the presence of GM-CSF and of a second cytokine that can be IL 4 (ref. 44) or IL 13 (ref. 49), plus TNF.

One of the aims of the invention is to provide cells with high phagocytosis, and efficient antigen presentation.

Another aim of the invention is to provide very potent antigen presenting cells derived from human blood monocytes.

Another aim of the invention is to provide cells presenting membrane receptors, allowing targeting and increased antigen presentation with the use of bispecific antibodies (refs. 27-31, 37).

Another aim of the invention is to provide cells which can be transfected with cDNA to be used in gene therapy (ref. 32 - 36).

Another aim of the invention is to provide a method allowing the recovery from human blood of cells of the macrophage lineage with high phagocytosis, digestive activity, processing MHC class I and class II antigen presentation (ref. 46, 50, 52).

Another aim of the invention is to provide a reproducible method allowing to obtain the above defined cells, said process not requiring combination of exogenous cytokines, defined media and recipients constituting a cell processor.

Another aim of the invention is to provide cellular vaccines demonstrating the high phagocytosis, processing, MHC-II peptide presentation of the macrophages and also the potent MHC-I T lymphocyte stimulation with high level of accessory molecules for presentation of the dendritic cells.

The invention relates to a process for preparing a composition comprising monocyte-derived antigen presenting cells (MD-APCs), said process comprising:
- starting from a composition containing leukocytes derived from healthy donors or from patients and obtained from peripheral blood by apheresis
- removal of platelets and anticoagulant from the apheresis product, such as by centrifugation of the apheresis products
- isolation of mononuclear cells (monocytes+lymphocytes) from red cells and granulocytes in order to have less than 10% granulocytes and less than 5% red cells,
- culture of mononuclear cells obtained at the previous stage by placing them in hydrophobic bags in an appropriate culture medium containing, as sole chemical ligands having receptors on the membrane of mononuclear cells:
- histamine or agonist of histamine receptor (H 1 in action) and a H2 antagonist
- or IL-13 in combination with "additional" GM-CSF,
for a time sufficient to obtain differentiated MD-APCs, preferably for about 5 to 15 days, and possibly separating the MD-APCs from the lymphocytes, and recovering the MD-APCs and lymphocytes.

The expression "additional" corresponds to the fact that there is no GM-CSF added to the culture in standard condition, but this does not exclude the fact that exogenous GM-CSF could be added in the culture to increase yield and functions of MD-APCs obtained.

The invention relates to macrophages which have the following properties :
- they present on their surface :
   * antigen CD14 with a mean intensity of about 20 to about 200,
   * antigen CD64 with a mean intensity of about 20 to about 200,
- they are substantially devoid of the surface antigens CD1a and CD1c, the presence and mean intensities respectively of CD14, CD64 and the absence of CD1a and CD1c being for instance determined by immunofluorescence staining and flow cytometry analysis,
- they present a phagocytosis property such as determined by the following test: said phagocytosis capacity being evaluated by an uptake of formalin fixed yeast, for example by culturing macrophages for 2 hours, adding yeast in 1/10 macrophage/yeast ratio and incubating at 37 °C, 5% CO₂ atmosphere for 2-3 hours fixing by the May-Grünwald-Giemsa (MGG) staining, and the percentage of phagocytic macrophages being quantified for instance by microscopic analysis,
- they have the property of stimulating the proliferation of allogenic lymphocytes such as determined by the following test :
   allogenic primary mixed lymphocytes reaction (MLR) was carried out in 96-well microtiter plates by adding increasing numbers (2x10³ to 2x10⁵) in 100 µl medium/well of macrophages to 2x10⁵ in 100 µl medium/well of allogenic T cells purified from buffy coats and after 5 days incubation at 37°C, cell proliferation was assessed by a colorimetric method, such as the hydrolysis of tetrazolium salt WST-1 (Boehringer Mannheim, Germany), (slightly red) to Formozan (dark red).

More generally, the invention relates to monocytes derived antigen presenting cells (MD-APCs), particularly macrophages which have the following properties :
- they present on their surface :
   * antigens CD14 and CD64 with a mean intensity of about 5 to about 200,
   * antigen CD80 and CD86 with a mean intensity of about 20 to about 200,
   * antigen CD40 and mannose receptor with a mean intensity of 50 to 500,
- they are substantially devoid of the surface antigens CD1a and CD1c, i.e. with a mean intensity lower than 20,
   the presence and mean intensities respectively of CD14, CD64, CD80 and CD86, and the absence of CD1a and CD1c being for instance determined by immunofluorescence staining and flow cytometry analysis,
- they present a phagocytosis property such as determined by the following test: said phagocytosis capacity being evaluated by an uptake of formalin fixed yeast, for example by culturing MD-APCs for 2 hours, adding yeast in 1/10 MD-APCs/yeast ratio and incubating at 37°C, 5% CO₂ atmosphere for 2-3 hours fixing by the May-Grünwald-Giemsa (MGG) staining, and the percentage of phagocytic MD-APCs being quantified for instance by microscopic analysis,
- they have the property of stimulating the proliferation of allogenic lymphocytes such as determined by the following test :
   allogenic primary mixed lymphocytes reaction (MLR) was carried out in 96-well microtiter plates by adding increasing numbers (2x10³ to 2x10⁵) in 100 *µ*l medium/well of MD-APCs to 2x10⁵ in 100 *µ*l medium/well of allogenic T cells purified from buffy coats and after 5 days incubation at 37°C, cell proliferation was assessed by measurement of Brdu incorporation during DNA synthesis by ELISA method (Boehringer Mannheim, Germany) or by a colorimetric method, such as the hydrolysis of tetrazolium salt WST-1 (Boehringer Mannheim, Germany), (slightly red) to Formozan (dark red).

In the context of the present invention, the expression "macrophages" designates not only macrophages but antigen presenting cells which derive from monocytes and which will be hereafter designated by MD-APC.

The expression "substantially devoid of surface antigens CD1a and CD1c" means either that there is no such surface antigens or that there is only a dim intensity for these surface antigens, with said dim intensity corresponding to about 10 times less than the intensity obtained in the presence of such surface antigens, as determined in immunofluorescence analysis, or with said intensity being lower than 20.

In the following of the text, it will be often referred to "the absence of surface antigen CD1a and CD1c", which is to be understood as "substantially devoid of such antigens" as explained above (intensity lower than 20).

The invention also relates to MD-APCs which present, on their surface, antigen MHC-II with a mean intensity of about 100 to about 600, such as determined by immunofluorescence staining and flow cytometry analysis.

The invention also relates to macrophages which are substantially devoid of surface antigen CD83, such as determined by immunofluorescence staining and flow cytometry analysis.

The expression "substantially devoid of surface antigen CD83" corresponds to an intensity lower than 20.

The MD-APCs of the invention present adherent properties such as determined by the following test :
the MD-APCs are cultured for 2 h in culture medium (I.M.D.M. or R.P.M.I.) on plastic flasks and the percentage (%) of adherent cells is quantified for instance by microscopic analysis.

The culture media I.M.D.M. and R.P.M.I. are commercially available.

The invention relates to a MD-APCs culture wherein :
- about 10% to about 50% of the MD-APCs present antigen CD14 on their surface,
- about 10% to about 50% of the MD-APCs present antigen CD64 on their surface,
- about 80% to about 100% of the MD-APCs present antigen MHC-II on their surface,
- about 70% to about 100% of the MD-APCs present adherent properties,
- about 30% to about 100% of the MD-APCs present antigens CD80 and CD86 in their surface,
- about 30 % to about 100 % of the MD-APCs present high phagocytosis property,
   each macrophage having the above-mentioned properties being such that said properties are expressed according to the intensities as specified above.

The invention also relates to a process for preparing a composition of macrophages which comprises the culture of mononuclear cells in a culture medium containing histamine or agonist of histamine receptor (H₁ in action) and a H₂ antagonist, in combination with "additional" GM-CSF.

The invention also relates to a process for preparing a composition of MD-APCs which comprises the culture of mononuclear cells in a culture medium containing a chemical ligand having receptors on the membrane of mononuclear cells, for example histamine or agonist of histamine receptor(H₁ in action) and a H₂ antagonist, in combination with "additional" GM-CSF.

An example of agonist of histamine receptor is 2 methyl-histamine.

As example of H₂ antagonist, one may cite cimetidine but also tiotidine, burimamide, metiamide, ranitidine.

As example of other chemical ligands interacting with mononuclear cells and allowing differentiation into MD-APCs, one may cite detoxified LPS such as lipid A, C3 and other ligands of complement receptors, taxols, oxydoreductors such as flavenoids or polyphenols, ligands to CD40, to the TNF receptors or to vitamin D3 receptors.

When the concentration of histamine or cimetidine or other chemical ligands for membrane receptors is lower than the lower value of the given range, there is substantially no effect, i.e. less than 10% difference with cells cultured in the absence of histamine and cimetidine.

When the concentration of histamine or cimetidine is higher than the higher value of the given range, the culture medium becomes toxic.

The invention also relates to a process wherein the culture medium contains a chemical ligand, for example histamine and cimetidine or a H₂ antagonist, in combination with "additional" GM-CSF, histamine being present at a concentration of about 10⁻² M to about 10⁻⁶ M, preferably of about 10⁻⁴ M, cimetidine or the H₂ antagonist being present at a concentration of about 10⁻⁴ M to about 10⁻⁹ M, preferably of about 10⁻⁶ M, and additional GM-CSF being present at a concentration of about 50 U/ml to about 1000 U/ml, preferably of about 500 U/ml.

The invention also relates to a process as above-defined, wherein the culture medium contains IL-13 in combination with "additional" GM-CSF.

When additional GM-CSF is incorporated into the culture, then the total concentration of GM-CSF is from about 50 U/ml to about 2000 U/ml, and preferably from about 50 U/ml to about 500 U/ml.

According to a particular embodiment of the invention, the culture medium does not comprise the following elements : exogenous cytokines such as IL4, IL10, TNF.

The invention also relates to a process comprising:
- isolation of leukocytes, from healthy donors or from patients, from peripheral blood by apheresis and removal of platelets and anticoagulant from the apheresis product,
- isolation of mononuclear cells (monocytes + lymphocytes) from red cells and granulocytes in order to have less than 10% granulocytes and less than 5% red cells,
- culture of the mononuclear cells obtained at the previous stage by placing them in an appropriate culture medium containing a chemical ligand of mononuclear cells, such as histamine or an agonist of histamine, an H₂ antagonist, such as cimetidine, in combination with GM-CSF, for a time sufficient to obtain differentiated MD-APCs, preferably for about 5 to 15 days, and possibly separating the MD-APCs from the lymphocytes, and recovering the MD-APCs or the MD-APCs and lymphocytes.
   The invention also relates to a process wherein the culture medium of MD-APCs is added
- with crude antigens, for instance autologous tumor membrane, killed tumoral cells, bacterial capsides, viral homogenates cleared from nucleic acids,
- specific peptides against which an immune response is desired,
- cDNA or genetic material linked to vectors (for example gluconated polylysine) to allow transfection of the macrophage with material coding for the relevant peptide or protein to be presented on the MD-APCs membrane and against which an immune response is desired,
- or bispecific antibodies targeting on the one side, a surface antigen or a surface receptor of the MD-APCs and, on the other side, a relevant antigen against which an immune response is desired.

The invention also relates to MD-APCs liable to be obtained according to the process of the invention described hereabove.

The invention also relates to pharmaceutical compositions containing as active substance, MD-APCs according to the invention.

The invention also relates to cellular vaccine compositions containing as active substance, MD-APCs according to the invention.

The invention also relates to the medium containing elements necessary for the growth and differentiation of monocytes into MD-APCs according to the invention, and in addition containing chemical ligands of mononuclear cells, such as histamine, cimetidine in combination with GM-CSF.

As example of other chemical ligands interacting with mononuclear cells and allowing differentiation into MD-APCs, one may cite detoxified LPS such as lipid A, C3 and other ligands of complement receptors, taxols, oxydoreductors susch as flavenoids or polyphenols, ligands to CD40, to the TNF receptors or to vitamin D3 receptors.

The invention also relates to a a cell processor or a kit containing :
- means for the recovery of lymphocytes and monocytes free of contaminants,
- appropriate buffer and wash solutions and possibly appropriate means for the conservation of macrophages,
- means for preparing a culture for the monocytes and possibly the lymphocytes and containing chemical ligands of mononuclear cells, for example histamine, cimetidine or a H₂ antagonist in combination with GM-CSF,
- possibly means for transfection of cultured cells and means for targeting antigens to MD-APCs.

Regarding the conservation of MD-APCs, it can include freezing means for example in 10% glycerol or D.M.S.O. (dimethyl sulfoxyde) in the presence of autologous or AB⁺ serum.

The invention also relates to a a cell processor or a kit as described above which contains :
- means for recovering and centrifuging blood to obtain a leukocyte concentrate,
- means for separating lymphocytes and monocytes from the other white cells and for eliminating the contaminating red cells,
- culture medium for MD-APCs and possibly lymphocytes with complements and particularly chemical ligands of mononuclear cells, such as histamine and cimetidine or a H₂ antagonist, in combination with GM-CSF,
- appropriate means for the conservation of macrophages,
- appropriate buffer and wash solution.

The invention also relates to products containing MD-APCs according to the invention, and lymphocytes, as a combined preparation for simultaneous, separate or sequential use in cell therapy.

The invention also relates to products as described hereabove which contain the MD-APCs and the lymphocytes in a ratio of at least 20% to 50% of MD-APCs expressed in cell number.

The invention also relates to bispecific antibodies liable to recognize an antigen of a MD-APCs of the invention and an antigen of a tumoral cell or of a pathogen which is to be targetted to said MD-APCs.

The invention also relates to a method for the clinical treatment, comprising the administration of an appropriate amount of MD-APCs according to the invention, and preferably in an amount of about 10⁸ to about 5x10⁹ MD-A PCs.

The invention also relates to a method for the treatment of any disorder, comprising the administration of lymphocytes from the culture in an amount of about 4x10⁹ to about 10x10⁹ lymphocytes.

The invention also relates to the use of a chemical ligands of mononuclear cells, such as an agonist of histamine receptor, in particular histamine, and a H₂ antagonist, in particular cimetidine, in combination with GM-CSF; for the preparation of MD-APCs having the following properties :
- they present on their surface :
   * antigens CD14 and CD64 with a mean intensity of about 5 to about 200,
   * antigen CD80 and CD86 with a mean intensity of about 20 to about 200,
   * antigen CD40 and mannose receptor with a mean intensity of 50 to 500,
- they are substantially devoid of the surface antigens CD1a and CD1c, i.e. with a mean intensity lower than 20,
   the presence and mean intensities respectively of CD14, CD64, CD80, CD86 and the absence of CD1a and CD1c being for instance determined by immunofluorescence staining and flow cytometry analysis,
- they present high phagocytosis property such as determined by the following test:
   said phagocytosis capacity being evaluated by an uptake of formalin fixed yeast, for example by culturing MD-APCs for 2 hours to select adherent cells, adding yeast in 1/10 macrophages to yeast ratio and incubating at 37°C, 5% CO₂ atmosphere for 2-3 hours fixing by the May-Grünwald-Giemsa (MGG) staining, and the percentage of phagocytic MD-APCs being quantified for instance by microscopic analysis,
- they have the property of stimulating the proliferation of allogenic lymphocytes such as determined by the following test :
   allogenic primary mixed lymphocytes reaction (MLR) was carried out in 96-well microtiter plates by adding different numbers 2x10³ to 2x10⁵ in 100 µl medium/well of MD-APCs to 2x10⁵ in 100 µl medium/well of allogenic T cells purified from buffy coats and after 5 days incubation at 37°C, cell proliferation was assessed by a colorimetric method, such as the cleavage of tetrazolium salt WST-1 (slightly red) to Formozan (dark red).

The monocytes derived antigens presenting cells of the invention and the MD-APCs can be obtained as follows :
a) Isolation of leukocytes from blood by apheresis and elimination of platelets, by centrifugation. If cells collected have < 10% granulocytes and haematocrit < 5%, they can be seeded as such in culture. Otherwise mononuclear cells have to be prepared first by centrifugation on Ficoll Paque of density 1,077.
b) Mononuclear cells are then cultured for 5 to 15 days in hydrophobic bags ethylene vinyl acetate (E.V.A., Stedim) or polypropylene (Life Cell, Baxter), at 37°C, 5% CO₂. Cells are seeded at 5.10⁶/ml in I.M.D.M. (as previous) or equivalent medium supplemented with indomethacin (5x10⁻⁶M) mercaptoethanol (3x10⁻⁵M), non essential amino-acids (1 %, Gibco) and 2 to 5% of reconstituted autologous *or AB*⁺ serum,
   with GM-CSF, 500 U/ml chemical ligands interacting with mononuclear cells and allowing differentiation into MD-APCs, such as detoxified LPS such as lipid A, C3 and other ligands of complement receptors, taxols, oxydoreductors susch as flavenoids or polyphenols, ligands to CD40, to the TNF receptors or to vitamin D3 receptors ; as example of ligands, histamine (10⁻⁴ M), cimetidine (10⁻⁶ M) or another H₂ antagonist of histamine, or with histamine, cimetidine in the absence of any exogenous cytokines. Endogenous cytokines are released by mononuclear cells stimulated by ligands.
   and exogenous antigens, peptides or transfectants (cDNA + vector) coding for the relevant antigen.
c) After culture, the specific monocyte derived antigen presenting cells (MD-APCs) are centrifuged, washed and resuspended for injection in the patient, to induce humoral and cellular immune response against the antigen.

The specificity of the cellular vaccine is achieved during the *in vitro* culture according to one of the following items.
a) culture of MD-APCs as explained above in b), in the presence of crude antigens, for example autologous tumor membrane, bacterial capsides, viral homogenates cleared from nucleic acids ;
b) culture of MD-APCs as explained above in b), in the presence of specific peptides against which immune response would be beneficial,
c) or culture of MD-APCs as explained above in b), in the presence of cDNA or genetic material linked to vectors (for example gluconated polyphysine) to allow transfection of MD-APCs with material coding for the relevant peptide or protein to be presented on the membrane.

In order to obtain specific cellular vaccine, it is also possible at the end of the differentiation stage of the macrophage culture to add bispecific antibodies targeting a membrane antigen, or a sugar receptor of MD-APCs on one side and the relevant antigen on the other side.

According to a preferred embodiment, the process of the invention comprises the following steps:
- isolation of leukocytes from blood of healthy subjects or patients by apheresis, to obtain the apheresis products (i.e. concentrated leukocytes),
- platelet elimination, for instance by centrifugation of the apheresis products, to obtain a leukocyte enriched product,
- separation, in the leukocyte enriched products, of the mononuclear cells on one hand, and of the contaminating red blood cells and granulocytes on the other hand,
- culture of the mononuclear cells (monocytes + lymphocytes) in a medium containing chemical ligands of mononuclear cells, such as histamine and cimetidine and GM-CSF for about 5 to 15 days, to obtain differentiated monocyte derived antigen presenting cells (MD-APCs).

The lymphocytes can be separated from the monocytes before the culture step.

The lymphocytes can be separated from the MD-APCs after the culture.

In the process of the invention, chemical ligands for mononuclear cells, such as histamine are used at a concentration of 10⁻² M to about 10⁻⁶ M, preferably of about 10⁻⁴ M.

In the process of the invention, GM-CSF is used at a concentration of about 50 to about 1000 U/ml, particularly of about 100 to about 500 U/ml.

In the process of the invention, the culture medium is RPMI, IMDM, MEM, or DMEM selected for very low endotoxin content.

These media are commercially available.

Advantageously, the culture medium contains indomethacin (or another cyclo-oxygenase inhibitor) or/and cimetidine (an histamine H₂ antagonist) and/or at its chemical ligands of mononuclear cells.

An advantageous process for preparing the MD-APCs of the invention is the following:

### Apheresis

Leukocytes from healthy subjects or from patients are isolated from peripheral blood by apheresis using the Cobe Spectra continous-flow blood cell separators keeping granulocytes contamination very low (< 10% and less than 5% red cells). The apheresis product is centrifuged for 10 min at 280 g in order to reduce platelet contamination. The platelet-enriched plasma is removed and leukocyte pellet resuspended in a phosphate buffer solution (PBS) containing 0.1% glucose, 0.17% PO₃HNa₂, 2H₂O, 0.27% PO₃H₂Na, 0.14% NH₄Cl, 0.78 % NaCl (solution TS745 laboratoire Bruneau, France).

The enriched leukocyte pellet is obtained with an average of 7 to 1.5x10¹⁰ leukocytes (50% of mononuclear cells).

### Isolation of mononuclear cells

If the collected leukocytes have more than 10% granulocytes contamination and/or 5% hematrocrite, human mononuclear cells are separated from red blood cells and from contaminating granulocytes, by 15 min centrifugation at 1000 g on a COBE 2991 or Stericell cell processor using Ficoll Paque of density 1.077 (Pharmacia). After 3 washings in phosphate buffered saline solution without calcium and magnesium, the monocytes are obtained with about 20% to 50% purity as shown by channelyser analysis (Coulter Margency-France).

### Culture

Differentiated human MD-APC are obtained by 5-15 days in culture of mononuclear cells in hydrophobic bags in E.V.A. (Ethylene Vinyl Acetate, STEDIM, Aubagne) or polypropylene (life cell-Baxter) at 37°C and 5% CO₂, 95% humidified atmosphere. Total mononuclear cells are seeded at 5x10⁶ cells/ml in Iscove modified medium (*I.M.D.M.,* Gibco) or equivalent medium supplemented by penicillin (100 UI/ml), streptomycine (100 µg/ml), L-glutamine (2 mM, Gibco), pyruvic acid (2 mM, Gibco), Indomethacin (5x10⁻⁶ M, Sigma), cimetidine (10⁻⁸ to 10⁻⁴ M), histamine (10⁻⁶ to 10⁻² M or other chemical ligands), mercaptoethanol (3x10⁻⁵ M, Gibco) non-essential amino-acids (1%, Gibco) and 2-5% of autologous or AB serum. The addition of GM-CSF (500 U/ml, SANDOZ) was done in comparative experiment.

According to a preferred embodiment, the process of the invention is such that killed tumoral cells are added into the culture medium simultaneously with monocytes, both cells coming preferably from the same patient, preferably at the ratio of about 1 million of killed tumoral cells/ml, with said killed tumoral cells being processed at the same time as macrophages.

The killed tumoral cells can then be processed simultaneously with the leukocytes, in an amount of about 1x10⁶/ml.

This process allows to obtain MD-APCs and lymphocytes specific for the tumor, inducing very efficiently *in vivo* an immune response to these specific tumor cells.

The invention also relates to MD-APCs liable to be obtained according to the above-defined process.

The invention also relates to pharmaceutical compositions containing, as active substance, MD-APCs as defined above.

The invention also relates to a medium containing elements necessary for the growth and differentiation of monocytes into MD-APCs of the invention, and in addition chemical ligands for mononuclear cells, for example histamine and GM-CSF.

The monocyte derived antigens presenting cells of the invention can be part of a cell processor or a kit containing:
- means for the recovery of lymphocytes and monocytes free of contaminants;
- appropriate buffer and wash solutions, and possibly appropriate means for the conservation of MD-APCs ;
- means for preparing a culture medium for the monocytes and possibly the lymphocytes and containing chemical ligands for mononuclear cells, for example histamine and/or GM-CSF.

According to an advantageous embodiment of the invention, the cell processor or kit contains :
- means for recovering and centrifuging blood to obtain a leukocyte concentrate;
- means for separating lymphocytes and monocytes from the other white cells and for eliminating the contaminating red cells;
- culture medium for MD-APCs and possibly lymphocytes with complements and particularly and/or GM-CSF and possibly indomethacin and/or cimetidine ;
- appropriate means for the conservation of the MD-APCs ;
- appropriate buffer and wash solutions ;

The invention also relates to products containing MD-APCs according to the invention, and lymphocytes, as a combined preparation for simultaneous, separate or sequential use in adoptive immunotherapy.

According to an advantageous embodiment, the products of the invention as defined above are characterised in that they contain the MD-APCs and the lymphocytes in a ratio of at least 20% to 50% of MD-APCs expressed in cell number.

In this embodiment, the MD-APCs and the lymphocytes are both injected to a patient.

The invention also relates to bispecific antibodies liable to recognize an antigen of a MD-APC of the invention, for example FCγRI (CD 64) and an antigen of a relevant antigen.

The bispecific antibodies can be prepared as described in Chokri et al. Res. Immunol. 143 (1992).

The bispecific antibodies can be injected at the same time as the MD-APCs of the invention, or can be pre-incubated with MD-APCs before injection.

The invention also relates to a method for the treatment of cancer and pathogens comprising the administration of an appropriate amount of MD-APCs according to the invention, and preferably in an amount of about 1x10⁸ to about 5x10⁹ MD-APCs.

### Description of the Figure:

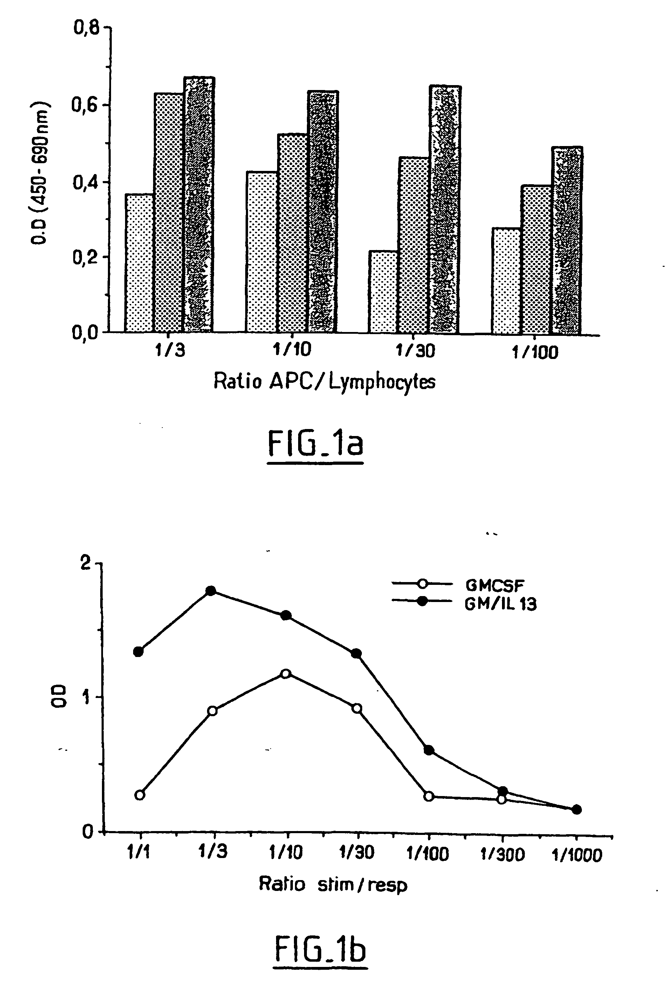

Figure 1a represents the allogenic T cell proliferation induced by MD-APCs of the invention recovered in the presence of histamine (10⁻⁴ M) and cimetidine (10⁻⁶ M) as adjuvant, with or without GM-CSF (500 U/ml) comparing to standard macrophages produced only in the presence of the GM-CSF (500 U/ml).
Figure 1b represents the stimulation by MD-APCs recovered in the presence of GM-CSF or GM-CSF + IL-13.

In Figure 1a, the optical density (450-690 nm) has been plotted against the ratio between the MD-APCs of the invention and the responder lymphocyte cells.

The clear dotted bars correspond to the presence of GM-CSF at 500 U/ml; the lighter grey bars correspond to the presence of histamine (10⁻⁴ M) and cimetidine (10⁻⁶ M).

The darker grey bars correspond to the presence of GM-CSF (500 U/ml), in combination with histamine (10⁻⁴ M) and cimetidine (10⁻⁶ M).

The results show that the capacity of the MD-APCs to stimulate the proliferation of allogenic lymphocytes is strongly increased. The stimulation of allogenic proliferation of lymphocytes by MD-APCs is very potent (at least 200% increase with respect to standard macrophages). The combination of histamine/cimetidine effect or IL-13 effect with GM-CSF can potentiate this activity (maximum of 300% increase with respect to the induction by macrophages).

In Figure 1b, the optical density (450-690 nm) has been plotted against the ratio between the MD-APCs of the invention and the responder lymphocyte cells. The curves with dark squares corresponds to the presence of GM-CSF/IL-13 and the curve with circles correspond to the presence of GM-CSF.

### EXAMPLE : Preparation of MD-APCs according to the invention :

### 1- Apheresis:

Leukocytes from healthy donors or from patients are isolated from peripheral blood by apheresis using COBE spectra blood cell separator keeping granulocytes contamination the lowest possible. The apheresis product is diluted in a phosphate buffered solution (PBS) (final volume = 450 ml). The apheresis product is centrifuged for 10 min at 280 g to remove platelets and anticoagulant.

### 2- Isolation of mononuclear cells:

If the collected cells have > 10% granulocytes contamination and/or > 5 % haematocrit, they should be centrifuged over Ficoll (density = 1.077) to remove red cells and granulocytes.

### 3- Culture

The monocyte derived antigen presenting cells (MD-APCs) are obtained after 5 to 15 days in culture of mononuclear cells in hydrophobic bags in EVA (Ethylene Vinyl Acetate /STEDIM) or equivalent bags (Teflon) at 37°C and 5% CO₂ in humidified atmosphere. The mononuclear cells are seeded at 5x10⁶ cells/ml in Iscove modified medium (I.M.D.M. - Gibco) or equivalent supplemented by (see PCT/EP93 page 7) the addition of ligands for mononuclear cells, such as histamine (10⁻⁴ M) or analogues and cimetidine (10⁻⁶ M) or similar H₂ antagonist, in the presence or not of GM-CSF (500 U/ml).

As example of other chemical ligands interacting with mononuclear cells and allowing differentiation into MD-APCs, one may cite detoxified LPS such as lipid A, C3 and other ligands of complement receptors, taxols, oxydoreductors susch as flavenoids or polyphenols, ligands to CD40, to the TNF receptors or to vitamin D3 receptors.

### 4- Characterization and functionality

a- Flow cytometry
   After the maturation of MD-APCs, the phenotype analysis of the obtained cells was performed by flow cytometry using murine FITC or P.E labelled monoclonal antibodies directed against membrane proteins.
b- Mixed lymphocytes reactions (MLR)
   To evaluate the induction of T cell response to MD-APCs, allogenic primary MLR was carried out in 96-Well microtiter plates by adding different numbers of MD-APCs to 2x10⁵ allogenic T cells purified from buffy coats. After 5 days at 37°C, cell proliferation was assessed by a colorimetric methods such as WST-1.
c- Phagocytosis
   The phagocytic capacity of the different cells obtained was evaluated by uptake of formalin fixed yeast. Briefly the cells were cultured for 2 hours to select adherent cells. The yeast was added at 1/10 macrophage/yeast ratio and incubated in 37°C, 5% CO₂ atmosphere for 2-3 h and then fixed by the May-Grünwald-Giemsa (MGG) staining. The percentage of phagocytic cells was quantified by microscopic analysis.

The results are gathered in table 1, table 2 and table 3.

**Table 1**

| Yield (% of cells) differentiated in culture | | |
|---|---|---|
| Time of culture | (a) Hist/Cim | (b) Hist.Cim/GM |
| Day 4 | 71% | 78% |
| Day 7 | 49% | 48% |
| Day 11 | 19% | 31 % |

Table 1 shows that there is a recovery of a large quantity of MD-APCs after 5 to 11 days of culture of mononuclear cells in hydrophobic bags, in the presence of histamine (10⁻⁴ M) and cimetidine (10⁻⁶ M) (a) and additional GM-CSF (500 U/ml) (b) as adjuvant. In comparison with production of mature macrophages in standard conditions, the recovery of MD-APC cells is in the same order.

**Table 2**

| Phenotype of MD-APCs produced under various conditions after 6 days of culture | | | | |
|---|---|---|---|---|
| Surface Ag | (a) Hist/Cim | | | (b) Hist.Cim/GM-CSF |
| | % | MIF | % | MIF |
| CD1a | N | | N | |
| CD1c | N | | N | |
| CD83 | N | | N | |
| CD 14 | 95 | 114 | 95 | 92 |
| CD 54 | 96 | 40 | 96 | 40 |
| CD 58 | 97 | 41 | 97 | 31 |
| CD 64 | 43 | 35 | 91 | 46 |
| HLA-DR | 92 | 350 | 96 | 400 |
| MIF = Mean Intensity Fluorescence. % = percentage of positive cells. N = Negative or weak signal. | | | | |

This table corresponds to the immunofluorescence profile analysis by flow cytometry of MD-APCs generated in culture (6 days) under different conditions. The cells obtained under histamine (10⁻⁴ M) and cimetidine (10⁻⁶ M) conditions (a) are positive for macrophage markers (CD14, CD64 and HLA-DR). The combination with GM-CSF (b) does not change the phenotypic profile of the MD-APCs. They also clearly express CD54 and CD58. The CD80 (B7.1) and CD86 (B7.2) are also expressed on their membrane but in lower level. In contrast, CD1a, CD1c and CD83 which are positive markers of dendritic cells, are weakly expressed by the MD-APCs.

From these data, it can be confirmed that the antigen presenting cells generated in the culture system of the invention are different from dendritic cells.

**Table 3**

| % of phagocytic cells after 3h contact with fixed yeast | | |
|---|---|---|
| Number of intracellular Yeast | (a) Hist/cim | (b) Hist.Cim/GM-CSF |
| 0 | 42% | 20% |
| 1-5 | 43.6% | 43% |
| 6-10 | 11% | 27.5% |
| > 10 | 3.4% | 9.5% |

The MD-APCs are tested for the phagocytic activity using formalin fixed yeast. After 3 h incubation and MGG (May Grümwald Giemsa) staining, the intracellular particles are quantified by microscopic analysis. The MD-APCs generated in the presence of histamine/cimetidine (a) present an important capacity of phagocytosis (60%) and this capacity is increased in the presence of GM-CSF in the culture (80%) (b).

From the above-mentioned results, it is shown that human mononuclear cells cultured in the presence of histamine and cimetidine, in combination or not with GM-CSF mainly for one week, differentiate into mature antigen presenting cells. During the culture, these cells acquire a high level of accessory functions like.

The results of the invention indicate that the MD-APCs obtained in the culture system of the invention are different from the dendritic cells (DC), since DC have been described as FC (CD64) receptor negative, poorly adherent and non-phagocytic cells, possessing only a small number of lysosomes.

In contrast the MD-APCs of the invention :
(a) show a high adherence capacity,
(b) show an important phagocytic and processing activity,
(c) express high level HLA-DR membrane antigens and a low level of CD1a and CD1c, which is strongly expressed by dendritic cells,
(d) are also positive for CD54, CD58, CD80 and CD86 membrane antigens,
(e) stimulate T-cell proliferation in allogenic MLR reaction, and this is in a far better way than macrophages obtained according to techniques of the prior art.

Table 4 hereafter gathers the comparative characterization of MD-APC of the invention on the one hand, and of dendritic cells on the other hand.

**Table 4**

| Comparative characterization of Antigen Presenting Cells: | | | | |
|---|---|---|---|---|
| Dendritic Cells | | MD-APC | | |
| | | % cells | Intensity | |
| Phenotype | | | | |
| CD1a | + | - | 0 | |
| CD1c | + | - | 0 | |
| CD 83 | + | - | 0 | |
| CD14 | +/- | + | 10 to 100 | 5 to 200 |
| CD64 | - | + | 10 to 100 | 5 to 200 |
| CMH-II | + + + | +++ | 80 to 100 | 100 to 400 |

| Functions: | | | | |
|---|---|---|---|---|
| Adherence | - | ++ | 70 to 90 % | |
| Phagocytosis | - | + + | 30 to 80 % | |
| Stimulation of MLR | +++ | ++ | | |

**Table 5**

| | | |
|---|---|---|
| It gathers a complete phenotypic characterization of MD-APCs recovered after 6 days of culture according to the invention (analysis by flow cytometry). | | |

| **Phenotype** | **% Cells** | **Mean fluo intensity** |
|---|---|---|
| | | |
| CD45 | 97,6 | |
| CD14 | 6,8 | 172 |
| CD3 | 13 | |
| CD19 | 15 | |
| CD56 | 3,8 | |
| | | |
| CD4-PE | 95 | |
| CD25 | 1,7 | |
| CD45RO | 99 | |
| | | |
| CD16 | 1,8 | 31 |
| CD32 | 63 | 163 |
| CD64 | 4 | 12 |
| | | |
| CD1a | 31 | 216 |
| CD1c | 58 | 505 |
| CD83 | 9 | 18 |
| | | |
| HLA-DR | 99 | 266 |
| HLA-I | 99,6 | 582 |
| CD40 | 98 | 991 |
| CD80 | 78 | 64 |
| CD86 | 99 | 744 |
| | | |
| IgG1-FITC | 6,7 | 11 |
| IgG1-PE | 20 | (16)55 |
| IgG1-Cy5 | 19 | (29)50 |
| IgG2a-FITC | 5,3 | 19 |
| | | |
| IgG1 i | 1,6 | 75 |
| IgG2a i | 3,8 | 21 |
| IgG2b i | 3,2 | 15 |

## Claims

1. A process for preparing a composition comprising monocyte-derived antigen presenting cells (MD-APCs), said process comprising:
- starting from a composition containing leukocytes derived from healthy donors or from patients and obtained from peripheral blood by apheresis
- removal of platelets and anticoagulant from the apheresis product, such as by centrifugation of the apheresis products
- isolation of mononuclear cells (monocytes+lymphocytes) from red cells and granulocytes in order to have less than 10% granulocytes and less than 5% red cells,
- culture of mononuclear cells obtained at the previous stage by placing them in hydrophobic bags in an appropriate culture medium containing, as sole chemical ligands having receptors on the membrane of mononuclear cells:
- histamine or agonist of histamine receptor (H1 in action) and a H2 antagonist
- or IL-13
in combination with additional exogenous GM-CSF,
for a time sufficient to obtain differentiated MD-APCs, preferably for about 5 to 15 days, and possibly separating the MD-APCs from the lymphocytes, and recovering the MD-APCs and lymphocytes.

2. A process according to claim 1, wherein removal of platelets and anticoagulant from the apheresis product is such as obtained by centrifugation of the apheresis products for 10 mn at 280g.

3. A process according to claims 1 or 2, wherein the culture medium contains
- histamine or agonist of histamine receptor (H1 in action)
- a H2 antagonist
in combination with additional exogenous GM-CSF.

4. A process according to claims 1 or 2, wherein the culture medium contains
IL-13
in combination with additional exogenous GM-CSF.

5. A process according to any one of claims 1 to 3, wherein the culture medium contains histamine and a H₂ antagonist, in combination with additional GM-CSF, histamine being present at a concentration of about 10⁻² M to about 10⁻⁶ M, and preferably of about 10⁻⁴ M, the H₂ antagonist being present at a concentration of about 10⁻⁴ M to about 10⁻⁹ M, and preferably of about 10⁻⁶ M, and additional GM-CSF being present at a concentration of about 50 U/ml to about 1000 U/ml, preferably of about 500 U/ml.

6. A process according to any of claims 1 to 3 or 5, wherein:
the agonist of histamine receptor (H₁ in action) is 2 methyl-histamine, and/or
the H₂ antagonist is chosen among the group consisting of: cimetidine, tiotidine, burinamide, metiamide and ranitidine.

7. Process according to any one of claims 1, 2 or 4, wherein the culture medium contains IL-13 and additional exogenous GM-CSF, said additional GM-CSF being present at a concentration of about 50 U/ml to about 1000 U/ml, and preferably of about 500 U/ml.

8. Process according to any one of claims 1 to 7, wherein the culture medium of MD-APCs is added with at least one of the following elements:
- crude antigens, for instance autologous tumor membrane, killed tumoral cells, bacterial capsides, viral homogenates cleared from nucleic acids,
- specific peptides against which an immune response is desired,
- cDNA or genetic material linked to vectors (for example gluconated polylysine) to allow transfection of the MD-APCs with material coding for the relevant peptide or protein to be presented on the MD-APC membrane and against which an immune response is desired,
- bispecific antibodies targeting on the one side, a surface antigen of the MD-APCs and, on the other side, a relevant antigen against which an immune response is desired.

9. Monocyte-derived antigen presenting cells (MD-APCs) obtained according to a process of any one of claims 1 to 8.

10. MD-APCs according to claim 9 which have the following properties:
- they present on their surface
* antigen CD14 and CD64 with a mean intensity of about 20 to about 200,
* antigen CD80 and CD86 with a mean intensity of about 20 to about 200,
* antigen CD40 and mannose receptor with a mean intensity of about 50 to about 500,
* antigens CD1a and CD1c with a mean intensity lower than 20,
the presence and mean intensities respectively of CD14, CD64, CD80, CD86, CD40, mannose receptor, CD1a and CD1c being determined by immunofluorescence staining and flow cytometry analysis,
- they present a phagocytosis property such as determined by the following test:
said phagocytosis capacity being evaluated by an uptake of formalin fixed yeast, by culturing macrophages for 2 hours, adding yeast in 1/10 macrophages/yeast ratio and incubating at 37°C, 5% CO₂ atmosphere for 2-3 hours fixing by the May-Grünwald-Giemsa (MGG) staining, and the percentage of phagocytic MD-APCs being quantified for instance by microscopic analysis,
- they have the property of stimulating the proliferation of allogenic lymphocytes such as determined by the following test:
allogenic primary mixed lymphocytes reaction (MLR) was carried out in 96-well microtiter plates by adding different numbers (2 × 10³ to 2 × 10⁵ in 100 µl medium/well) of MD-APCs to 2 × 10⁵ in 100 µl medium/well of allogenic T cells purified from buffy coats and after 5 days incubation at 37°C, cell proliferation was assessed by a colorimetric method, such as the hydrolysis of tetrazolium salt WST-1 (Boehringer Mannheim, Germany), (slightly red) to Formozan (dark red).

11. MD-APCs according to claim 9 or 10, presenting a capacity of phagocytosis of 80%.

12. MD-APCs according to any one of claims 9 to 11, which present, on their surface, antigen MHC-II with a mean intensity of about 100 to about 400, such as determined by immunofluorescence staining and flow cytometry analysis.

13. MD-APCs according to any one of claims 9 to 12, which are substantially devoid of surface antigen CD83, such as determined by immunofluorescence staining and flow cytometry analysis.

14. MD-APCs according to any one of claims 9 to 13, which present adherent properties such as determined by the following test:
the macrophages are cultured for 2 h in culture medium (I.M.D.M. or R.P.M.I.) on plastic flasks and the percentage (%) of adherent cells is quantified for instance by microscopic analysis.

15. Monocyte-derived antigen presenting cells (MD-APCs) which have the following properties:
- they present on their surface:
* antigen CD14 and CD64 with a mean intensity of about 20 to about 200,
* antigen CD80 and CD86 with a mean intensity of about 20 to about 200,
* antigen CD40 and mannose receptor with a mean intensity of 50 to 500,
* antigens CD1a and CD1c, with a mean intensity lower than 20,
the presence and mean intensities respectively of CD 14, CD64, CD80, CD86, CD40, mannose receptor, CD1a and CD1c being determined by immunofluorescence staining and flow cytometry analysis,
- they present a phagocytosis property such as determined by the following test:
said phagocytosis capacity being evaluated by an uptake of formalin fixed yeast, by culturing macrophages for 2 hours, adding yeast in 1/10 macrophages/yeast ratio and incubating at 37°C, 5% CO₂ atmosphere for 2-3 hours fixing by the May-Grünwald-Giemsa (MGG) staining, and the percentage of phagocytic MD-APCs being quantified for instance by microscopic analysis,
- they have the property of stimulating the proliferation of allogenic lymphocytes such as determined by the following test:
allogenic primary mixed lymphocytes reaction (MLR) was carried out in 96-well microtiter plates by adding different numbers (2 × 10³ to 2 × 10⁵ in 100 *µ*l medium/well) of MD-APCs to 2 × 10⁵ in 100 *µ*l medium/well of allogenic T cells purified from buffy coats and after 5 days incubation at 37°C, cell proliferation was assessed by a colorimetric method, such as the hydrolysis of tetrazolium salt WST-1 (Boehringer Mannheim, Germany), (slightly red) to Formozan (dark red).

16. MD-APCs according to claim 15, presenting a capacity of phagocytosis of 80%.

17. MD-APCs according to claim 15 or 16, which present, on their surface, antigen MHC-II with a mean intensity of about 100 to about 400, such as determined by immunofluorescence staining and flow cytometry analysis.

18. MD-APCs according to any one of claims 15 to 17, which are substantially devoid of surface antigen CD83, such as determined by immunofluorescence staining and flow cytometry analysis.

19. MD-APCs according to any one of claims 15 to 18, which present adherent properties such as determined by the following test:
the macrophages are cultured for 2 h in culture medium (I.M.D.M. or R.P.M.I.) on plastic flasks and the percentage (%) of adherent cells is quantified for instance by microscopic analysis.

20. Pharmaceutical compositions containing as active substance, MD-APCs according to any one of claims 9 to 19.

21. Cellular vaccine compositions containing as active substance, MD-APCs according to any one of claims 9 to 19.

22. Medium containing elements necessary for the growth and differentiation of monocytes into MD-APCs according to a process of any one of claims 1 to 8, containing as sole chemical ligands having receptors on the membrane of mononuclear cells:
- histamine or agonist of histamine receptor (H₁ in action), and a H₂ antagonist such as cimetidine,
- or IL-13,
in combination with additional exogenous GM-CSF.

23. Medium according to claim 21, containing:
- histamine or an agonist of histamine receptor (H₁ in action), and
- a H₂ antagonist, and
additional exogenous GM-CSF.

24. Medium according to any one of claims 21 to 23, containing histamine and an H₂ antagonist, in combination with additional exogenous GM-CSF, histamine being present at a concentration of about 10⁻² M to about 10⁻⁶ M, and preferably of about 10⁻⁴ M, the H₂ antagonist being present at a concentration of about 10⁻⁴ M to about 10⁻⁹ M, and preferably of about 10⁻⁶ M, and additional exogenous GM-CSF being present at a concentration of about 50 U/ml to about 1000 U/ml, preferably of about 500 U/ml.

25. Medium according to any of claims 21 to 24, wherein:
- the agonist of histamine receptor (H₁ in action) is 2 methyl-histamine, and/or
- the H₂ antagonist is chosen among the group consisting of: cimetidine, tiotidine, burinamide, metiamide and ranitidine.

26. Medium according to claim 25, wherein the H₂ antagonist is cimetidine.

27. Medium according to claim 22, wherein IL-13 and GM-CSF are added in the culture medium, additional exogenous GM-CSF being present at a concentration of about 50 U/ml to about 1000 U/ml, and preferably of about 500 U/ml.

28. Cell processor or kit containing:
- means for the recovery of lymphocytes and monocytes free of contaminants,
- appropriate buffer and wash solutions and possibly appropriate means for the conservation of monocyte-derived antigen presenting cells (MD-APCs),
- means for preparing a culture for the monocytes and possibly the lymphocytes and containing as sole chemical ligands having receptors on the membrane of mononuclear cells
- histamine or an agonist of histamine receptor (H₁ in action), and a H₂ antagonist such as cimetidine,
- or IL-13,
in combination with GM-CSF,
- possibly means for transfection of cultured cells and means for targeting antigens to MD-APCs.

29. Cell processor or kit according to claim 28, containing:
- histamine or an agonist of histamine receptor (H₁ in action), and
- a H₂ antagonist,
in combination with GM-CSF.

30. Cell processor or kit according to claim 28, containing IL-13 and GM-CSF.

31. Cell processor or kit according to any one of claim 26 to 28, containing means for preparing a culture for the monocytes and possibly the lymphocytes, and containing histamine and cimetidine.

32. Cell processor or kit according to claim 30, containing means for preparing a culture for the monocytes and possibly the lymphocytes, and containing IL-13 and GM-CSF.

33. Cell processor or kit according to any of claims 28 to 32, containing:
- means for recovering and centrifuging blood to obtain a leukocyte concentrate,
- means for separating lymphocytes and monocytes from the other white cells and for eliminating the contaminating red cells,
- culture medium for MD-APCs and possibly lymphocytes with complements and particularly, as sole chemical ligands having receptors on the membrane of mononuclear cells
histamine or an agonist of histamine receptor (H₁ in action), and a H₂ antagonist such as cimetidine,
or IL-13,
in combination with GM-CSF,
- appropriate means for the conservation of MD-APCs,
- appropriate buffer and wash solution.

34. Cell processor or kit according to claim 28, containing culture medium for MD-APCs and possibly lymphocytes, with complements being histamine and cimetidine, in combination with GM-CSF.

35. Cell processor or kit according to claim 27, containing culture medium for MD-APCs and possibly lymphocytes, with complements being IL-13 and GM-CSF.

36. Products containing MD-APCs according to any one of claims 9 to 17, and lymphocytes, as a combined preparation for simultaneous, separate or sequential use in cell therapy.

37. Products according to claim 36, **characterized in that** they contain the MD-APCs and the lymphocytes in a ratio of at least 20% to 50% of MD-APCs expressed in cell number.

38. Use of MD-APCs according to any of claims 9 to 17, for the preparation of a drug for the treatment of cancer and of infections by pathogens.

39. Use of MD-APCs according to any of claims 9 to 17, for the preparation of a drug for the treatment of a disease involving phagocytosis, and the stimulation of the proliferation of T-lymphocytes.

40. Use according to claim 38 or 39, **characterized in that** said drug contains from about 10⁸ to about 5 × 10⁹ MD-APCs.

41. Use according to any one of claims 38 to 40, **characterized in that** said drug also contains lymphocytes.

42. Use of histamine or an agonist of histamine receptor (H₁ in action), and a H₂ antagonist, in particular cimetidine, or of IL-13, in combination with GM-CSF, for the in vitro preparation of monocyte-derived antigen presenting cells (MD-APCs) having the following properties:
- they present on their surface:
antigen CD 14 and CD64 with a mean intensity of about 20 to about 200,
antigen CD80 and CD86 with a mean intensity of about 20 to about 200,
antigen CD40 and mannose receptor with a mean intensity of 50 to 500,
CD1a and CD1c with a mean intensity larger than 20, the presence and mean intensities respectively of CD14, CD64, CD80, CD86, CD40, mannose receptor,
CD1a and CD1c being determined by immunofluorescence staining and flow cytometry analysis,
- they present high phagocytosis property such as determined by the following test:
said phagocytosis capacity being evaluated by an uptake of formalin fixed yeast, by culturing MD-APCs for 2 hours to select adherent cells, adding yeast in 1/10 mMD-APCs/yeast ratio and incubating at 37°C, 5% CO₂ atmosphere for 2-3 hours fixing by the May-Grünwald-Giemsa (MGG) staining, and the percentage of phagocytic MD-APCs being quantified for instance by microscopic analysis,
- they have the property of stimulating the proliferation of allogenic lymphocytes such as determined by the following test:
allogenic primary mixed lymphocytes reaction (MLR) was carried out in 96-well microtiter plates by adding different numbers (2 × 10³ to 2 × 10⁵ in 100 *µ*l medium/well) of MD-APCs to 2 × 10⁵ in 100 *µ*l medium/well of allogenic T cells purified from buffy coats and after 5 days incubation at 37°C, cell proliferation was assessed by a colorimetric method, such as the cleavage of tetrazolium salt WST-1 (slightly red) to Formozan (dark red) or such as Brdu incorporation during DNA synthesis.

## Patentansprüche

1. Verfahren zur Herstellung einer Zusammensetzung, die Monozyten-abgeleitete antigenpräsentierende Zellen (MD-APCs) aufweist, wobei das Verfahren umfasst:
- Ausgehen von einer Zusammensetzung, die Leukozyten enthält, die von gesunden Spender oder von Patienten stammen, und die aus peripherem Blut durch Apherese erhalten wurden,
- Entfernen von Blutplättchen und Antikoagulans aus dem Aphereseprodukt, wie durch Zentrifugieren des Aphereseproduktes,
- Isolieren mononukleärer Zellen (Monozyten + Lymphozyten) von roten Blutkörperchen und Granulozyten, um weniger als 10% Granulozyten und weniger als 5% rote Blutkörperchen zu haben,
- Kultur der im vorigen Schritt erhaltenen mononukleären Zellen, indem sie in hydrophoben Beuteln in ein geeignetes Kulturmedium gebracht werden, das als einzige chemische Liganden, die Rezeptoren an der Membran mononukleärer Zellen haben:
- Histamin oder Agonist des Histamin-Rezeptors (H1 in Aktion) und einen H2-Antagonisten
- oder IL-13
in Kombination mit zusätzlichem exogenen GM-CSF enthält,
für eine Zeitdauer, die ausreichend ist, um differenzierte MD-APCs zu erhalten, vorzugsweise etwa 5 bis 15 Tage, und gegebenenfalls Abtrennung der MD-APCs von den Lymphozyten und Gewinnung der MD-APCs und Lymphozyten.

2. Verfahren nach Anspruch 1, wobei die Entfernung der Blutplättchen und des Antikoagulans aus dem Aphereseprodukt so ist, wie sie durch Zentrifugieren des Aphereseproduktes für 10 min bei 280 g erhalten wird.

3. Verfahren nach den Ansprüchec 1 oder 2, wobei das Kulturmedium
- Histamin oder einen Agonisten des Histamin-Rezeptors (H1 in Aktion)
- einen H₂-Antagonisten
in Kombination mit zusätzlichem exogenen GM-CSF enthält.

4. Verfahren nach den Ansprüchen 1 oder 2, wobei das Kulturmedium IL-13 in Kombination mit zusätzlichem exogenen GM-CSF enthält.

5. Verfahren nach einem der Ansprüche 1 bis 3, wobei das Kulturmedium Histamin und einen H₂-Antagonisten in Kombination mit zusätzlichem GM-CSF enthält, wobei Histamin in einer Konzentration von etwa 10⁻² M bis etwa 10⁻⁶ M, vorzugsweise etwa 10⁻⁴ M enthalten ist, der H₂-Antagonist in einer Konzentration von etwa 10⁻⁴ M bis etwa 10⁻⁹ M, vorzugsweise etwa 10⁻⁶ M enthalten ist und zusätzliches GM-CSF in einer Konzentration von etwa 50 U/ml bis etwa 1000 U/ml, vorzugsweise etwa 500 U/ml enthalten ist.

6. Verfahren nach einem der Ansprüche 1 bis 3 oder 5, worin:
- der Agonist des Histamin-Rezeptors (H₁ in Aktion) 2-Methylhistamin ist und/oder
- der H₂-Antagonist ausgewählt ist aus der Gruppe bestehend aus Cimetidin, Tiotidin, Burinamid, Metiamid und Ranitidin.

7. Verfahren nach einem der Ansprüche 1, 2 oder 4, worin das Kulturmedium IL-13 und zusätzliches exogenes GM-CSF enthält, wobei das zusätzliche GM-CSF in einer Konzentration von etwa 50 U/ml bis etwa 1000 U/ml, vorzugsweise etwa 500 U/ml enthalten ist.

8. Verfahren nach einem der Ansprüche 1 bis 7, wobei dem Kulturmedium der MD-APCs mindestens eines der folgenden Elemente zugesetzt wird:
- rohe Antigene, beispielsweise autologe Tumormembran, abgetötete Tumorzellen, bakterielle Capside, virale Homogenate, die von Nukleinsäuren gereinigt sind,
- spezifische Peptide, gegen die eine Immunantwort erwünscht ist,
- cDNA oder genetisches Material, das mit Vektoren verknüpft ist (z.B. gluconiertes Polylysin), um die Transfektion der MD-APCs mit Material zu ermöglichen, das für das relevante Peptid oder Protein codiert, das an der MD-APC-Membran präsentiert werden soll und gegen das eine Immunantwort erwünscht ist,
- bispezifische Antikörper, die auf der einen Seite auf ein Oberflächenantigen der MD-APCs, auf der anderen Seite auf ein relevantes Antigen, gegen das eine Immunantwort erwünscht ist, zielen.

9. Monozyten-abgeleitete antigenpräscnticrende Zellen (MD-APCs), die nach einem Verfahren nach einem der Ansprüche 1 bis 8 erhalten wurden.

10. MD-APCs nach Anspruch 9 mit den folgenden Eigenschaften:
- sie präsentieren an ihrer Oberfläche:
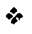 Antigen CD14 und CD64 mit einer mittleren Intensität von etwa 20 bis etwa 200
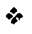 Antigen CD80 und CD86 mit einer mittleren Intensität von etwa 20 bis etwa 200,
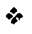 Antigen CD40 und Mannoserezeptor mit einer mittleren Intensität von etwa 50 bis etwa 500,
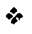 Antigene CD1a und CD1c mit einer mittleren Intensität von weniger als 20,
wobei das Vorhandensein bzw. die mittleren Intensitäten von CD14, CD64, CD80, CD86, CD40, Mannoserezeptor, CD1a und CD1c durch Immunfluoreszenz-Färbung und Durchflusszytometrie-Analyse bestimmt werden,
- sie zeigen eine Phagezytose-Eigenschaft, wie sie durch den folgenden Test bestimmt wird:
die Phagozytosekapazität wird bestimmt durch Aufnahme von Formalinfixierter Hefe, durch 2-stündiges Kultivieren von Makrophagen, Zugabe von Hefe in einem Verhältnis von 1/10 Makrophagen/Hefe und Inkubieren bei 37°C, 5%-CO₂-Atmosphäre während 2-3 Stunden, Fixieren durch die May-Grünwald-Giemsa (MGG)-Färbung, und quantitative Bestimmung des Prozentsatzes phagozylischer MD-APCs beispielsweise durch mikroskopische Analyse,
- sie haben die Eigenschaft, die Proliferation allogener Lymphozyten zu stimulieren, wie durch den folgenden Test bestimmt:
allogene primäre Lyraphozyten-Mischreaktion (MLR) wurde in Mikrotiterplatten mit 96 Vertiefungen durchgeführt, indem verschiedene Anzahl MD-APCs (2 × 10³ bis 2 × 10⁵ in 100 µl Medium/Vertiefung) zu 2 × 10⁵ allogenen T-Zellen, die vom Buffy coat gereinigt wurden, in 100 µl Medium/Vertiefung zugegeben wurden und nach 5-tägiger Inkubation bei 37°C die Zellproliferation nach einem kolorimetrischen Verfahren, wie Hydrolyse von Tetrazoliumsalz WST-1 (Boehringer Mannheim, Deutschland)(leicht rot) zu Formozan (dunkelrot), bestimmt wurde.

11. MD-APCs nach Anspruch 9 oder 10, die eine Phagozytosekapazität von 80% zeigen.

12. MD-APCs nach einem der Ansprüche 9 bis 11, die an ihrer Oberfläche Antigen MHC-II mit einer mittleren Intensität von etwa 100 bis etwa 400 präsentieren, wie durch immunfluoreszenzfärbung und Durchflusszytometrie-Analyse bestimmt.

13. MD-APCs nach einem der Ansprüche 9 bis 12, die im Wesentlichen kein Oberflächenantigen CD83 ausweisen, wie durch Immunfluoreszenzfärbung und Durchflusszytometrie-Analyse bestimmt.

14. MD-APCs nach einem der Ansprüche 9 bis 13, die Adhäsionseigenschaften zeigen, wie sie durch den folgenden Test bestimmt werden:
2-stündiges Kultivieren der Makrophagen in einem Kulturmedium (I.M.D.M. oder R.P.M.I.) an Kunststoffkolben und quantitative Bestimmung des Prozentsatzes (%) der adhärenten Zellen beispielsweise durch mikroskopische Analyse.

15. Monozyten-abgeleitete antigenpräsentierenden Zellen (MD-APCs) mit den folgenden Eigenschaften:
- sie präsentieren an ihrer Oberfläche:
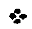 Antigen CD14 und CD64 mit einer mittleren Intensität von etwa 20 bis etwa 200,
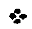 Antigen CD80 und CD86 mit einer mittleren Intensität von etwa 20 bis etwa 200,
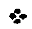 Antigen CD40 und Mannoserezeptor mit einer mittleren Intensität von etwa 50 bis etwa 500,
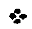 Antigene CD1a und CD1c mit einer mittleren Intensität von weniger als 20,
wobei das Vorhandensein bzw. die mittleren Intensitäten von CD14, CD64, CD80, CD86, CD40, Mannoserezeptor, CD1a und CD1c durch Immunfluoreszenz-Färbung und Durchflusszytometrie-Analyse bestimmt werden,
- sie zeigen eine Phagozytose-Eigenschaft, wie sie durch den folgenden Test bestimmt wird:
die Phagozytosekapazität wird bestimmt durch Aufnahme von Formalinfixierter Hefe, durch 2-stündiges Kultivieren von Makrophagen, Zugabe von Hefe in einem Verhältnis von 1/10 Makrophagen/Hefe und Inkubieren bei 37°C, 5%-CO₂-Atmosphäre während 2-3 Stunden, Fixieren durch die May-Grünwald-Giemsa (MGG)-Färbung, und quantitative Bestimmung des Prozentsatzes phagozytischer MD-APCs beispielsweise durch mikroskopische Analyse,
- sie haben die Eigenschaft, die Proliferation allogener Lymphozyten zu stimulieren, wie durch den folgenden Test bestimmt:
allogene primäre Lymphozyten-Mischreaktion (MLR) wurde in Mikrotiterplatten mit 96 Vertiefungen durchgeführt, indem verschiedene Anzahl MD-APCs (2 × 10³ bis 2 × 10⁵ in 100 µl Medium/Vertiefung) zu 2 × 10⁵ allogenen T-Zellen, die vom Buffy coat gereinigt wurden, in 100 µl Medium/Vertiefung zugegeben wurden und nach 5-tägiger Inkubation bei 37°C die Zellproliferation nach einem kolorimetrischen Verfahren, wie Hydrolyse von Tetrazoliumsalz WST-1 (Boehringer Mannheim, Deutschland) (leicht rot) zu Formozan (dunkelrot), bestimmt wurde.

16. MD-APCs nach Anspruch 15, die eine Phagozytosckapazität von 80 % zeigen.

17. MD-APCs nach einem der Ansprüche 15 oder 16, die an ihrer Oberfläche Antigen MHC-II mit einer mittleren Intensität von etwa 100 bis etwa 400 präsentieren, wie durch Immunfluoreszenzfärbung und Durchflusszytometrie-Analyse bestimmt.

18. MD-APCs nach einem der Ansprüche 15 bis 17, die im Wesentlichen kein Oberflächenantigen CD83 aufweisen, wie durch Immunfluoreszenzfärbung und Durchflusszytometrie-Analyse bestimmt.

19. MD-APCs nach einem der Ansprüche 15 bis 18, die Adhäsionseigenschaften zeigen, wie sie durch folgenden Test bestimmt werden:
2-stündiges Kultivieren der Makrophagen in einem Kulturmedium (I.M.D.M. oder R.P.M.L) an Kunststoffkolben und quantitative Bestimmung des Prozentsatzes (%) adhärenter Zellen beispielsweise durch mikroskopische Analyse.

20. Pharmazeutische Zusammensetzungen, die als Wirkstoff MD-APCs nach einem der Ansprüche 9 bis 19 enthalten.

21. Zelluläre Vakzin-Zusammensetzungen, die als Wirkstoff MD-APCs nach einem der Ansprüche 9 bis 19 enthalten.

22. Medium, das die für das Wachstum und die Differenzierung der Monozyten in MD-AFCs gemäß einem Verfahren nach einem der Ansprüche 1 bis 8 erforderlichen Elemente aufweist, das als einzige chemische Liganden, die Rezeptoren an der Membran mononukleärer Zellen haben,
- Histamin oder Agonist des Histamin-Rezeptors (H₁ in Aktion) und einen H₂-Antagonisten wie Cimetidin,
- oder IL-13
in Kombination mit zusätzlichem exogenen GM-CSF aufweist.

23. Medium nach Anspruch 21, das
- Histamin oder einen Agonisten des Histamin-Rezeptors (H₁ in Aktion) und
- einen H₂-Antagonisten und
zusätzliches exogenes GM-CSF enthält.

24. Medium nach einem der Ansprüche 21 bis 23, das Histamin und einen H₂-Antagonisten in Kombination mit zusätzlichem exogenen GM-CSF enthält, wobei Histamin in einer Konzentration von etwa 10⁻² M bis etwa 10⁻⁶ M, vorzugsweise etwa 10⁻⁴ M enthalten ist, der H₂-Antagonist in einer Konzentration von etwa 10⁻⁴ M bis etwa 10⁻⁹ M, vorzugsweise etwa 10⁻⁶ M enthalten ist und zusätzliches exogenes GM-CSF in einer Konzentration von etwa 50 U/ml bis etwa 1000 U/ml, vorzugsweise etwa 500 U/ml enthalten ist.

25. Medium nach einem der Ansprüche 21 bis 24, worin:
- der Agonist des Histamin-Rezeptors (H₁ in Aktion) 2-Methylhistamin ist und/oder
- der H₂-Antagonist ausgewählt ist aus der Gruppe bestehend aus Cimetidin, Tiotidin, Burinamid, Metiamid und Ranitidin.

26. Medium nach Anspruch 25, worin der H₂-Antagonist Cimetidin ist.

27. Medium nach Anspruch 22, worin dem Kulturmedium IL-13 und GM-CSF zugesetzt sind, wobei das zusätzliche exogene GM-CSF in einer Konzentration von etwa 50 U/ml bis etwa 1000 U/ml, vorzugsweise etwa 500 U/ml enthalten ist.

28. Zellprozessor oder Kit, enthaltend:
- Mittel, um Lymphozyten und Monozyten frei von Verunreinigungen zu gewinnen,
- geeignete Puffer- und Waschlösungen und gegebenenfalls geeignete Mittel zur Konservierung Monozyten-abgeleiteter antigenpräsentierender Zellen (MD-APCs),
- Mittel zum Bereiten einer Kultur für die Monozyten und gegebenenfalls die Lymphozyten, das als einzige chemische Liganden, die Rezeptoren an der Membran mononukleärer Zellen haben,
- Histamin oder einen Agonisten des Histamin-Rezeptors (H₁ in Aktion) und einen H₂-Antagonisten, wie Cimetidin,
- oder IL-13
in Kombination mit GM-CSF, enthält,
- gegebenenfalls Mittel zur Transfektion kultivierter Zellen und Mittel zum Targeting von Antigenen zu MD-APCs.

29. Zellprocessor oder Kit nach Anspruch 28, enthaltend:
- Histamin oder einen Agonisten des Histamin-Rezeptors (H₁ in Aktion) und
- einen H₂-Antagonisten
in Kombination mit GM-CSF.

30. Zellprozessor oder Kit nach Anspruch 28, IL-13 und GM-CSF enthaltend.

31. Zellprozessor oder Kit nach einem der Ansprüche 26 bis 28, Mittel zum Bereiten einer Kultur für die Monozyten und gegebenenfalls die Leukozyten enthaltend und Histazmin und Cimetidin enthaltend.

32. Zellprozessor oder Kit nach Anspruch 30, Mittel zum Bereiten einer Kultur für die Monozyten und gegebenenfalls die Leukozyten enthaltend und IL-13 und GM-CSF enthaltend.

33. Zellprozessor oder Kit nach einem der Ansprüche 28 bis 32, enthaltend:
- Mittel zum Gewinnen und Zentrifugieren von Blut, um ein Leukozytenkonzentrat zu erhalten,
- Mittel zum Abtrennen von Lymphozyten und Monozyten von den anderen weißen Blutkörperchen und zum Eliminieren der verunreinigenden roten Blutkörperchen,
- Kulturmedium für MD-APCs und gegebenenfalls Lymphozyten mit Komplementen und insbesondere als einzige chemische Liganden, die Rezeptoren an der Membran mononukleärer Zellen haben,
Histamin oder einen Agonisten des Histamin-Rezeptors (H₁ in Aktion) und einen H₂-Antagonisten, wie Cimetidin,
oder IL-13,
in Kombination mit GM-CSF,
- geeignete Mittel zum Konservieren von MD-APCs,
- geeignete Puffer- und Waschlösung.

34. Zellprozessor oder Kit nach Anspruch 28, Kulturmedium für MD-APCs und gegebenenfalls Lymphozyten enthaltend, wobei die Komplemente Histamin und Cimetidin sind, in Kombination mit GM-CSF.

35. Zellprozessor oder Kit nach Anspruch 27, Kulturmedium für MD-APCs und gegebenenfalls Lymphozyten enthaltend, wobei die Komplemente IL-13 und GM-CSF sind.

36. Produkte, die MD-APCs nach einem der Ansprüche 9 bis 17 und Lymphozyten enthalten, als kombinierte Zubereitung für die gleichzeitige, getrennte oder aufeinander folgende Verwendung in der Zelltherapie.

37. Produkte nach Anspruch 36, **dadurch gekennzeichnet, dass** sie die MD-APCs und die Lymphozyten in einem Verhältnis von mindestens 20% bis 50% MD-APCs, ausgedrückt durch die Zellzahl, enthalten.

38. Verwendung von MD-APCs nach einem der Ansprüche 9 bis 17 für die Herstellung eines Medikaments zur Behandlung von Krebs und Infektionen durch Pathogene.

39. Verwendung von MD-APCs nach einem der Ansprüche 9 bis 17 zur Herstellung eines Medikaments zur Behandlung einer Erkrankung, die Phagozytose involviert, und Stimulierung der Proliferation von T-Lymphozyten.

40. Verwendung nach Anspruch 38 oder 39, **dadurch gekennzeichnet, dass** das Medikament etwa 10⁸ bis etwa 5 × 10⁹ MD-APCs enthält.

41. Verwendung nach einem der Ansprüche 38 bis 40, **dadurch gekennzeichnet, dass** das Medikament auch Lymphozyten enthält.

42. Verwendung von Histamin oder eines Agonisten des Histamin-Rezeptors (H₁ in Aktion) und eines H₂-Antagonisten, insbesondere Cimetidin, oder von IL-13, in Kombination mit GM-CSF, zur *in vitro* Herstellung von Monozylen-abgeleiteten antigenpräsentierenden Zellen (MD-APCs) mit den folgenden Eigenschaften:
- sie präsentieren an ihrer Oberfläche:
Antigen CD 14 und CD64 mit einer mittleren Intensität von etwa 20 bis etwa 200,
Antigen CD80 und CD86 mit einer mittleren Intensität von etwa 20 bis etwa 200,
Antigen CD40 und Mannoserezeptor mit einer mittleren Intensität von 50 bis 500,
CD1a und CD1c mit einer mittleren Intensität von weniger als 20,
wobei das Vorhandensein bzw. die mittleren Intensitäten von CD14, CD64, CD80, CD86, CD40, Mannoserezeptor, CD1a und CD1c durch Immunfluoreszenz-Färbung und Durchflusszytometrie-Analyse bestimmt werden,
- sie zeigen hohe Phagozytose-Eigenschaft, wie sie durch den folgenden Test bestimmt wird:
die Phagozytosekapazität wird bestimmt durch Aufnahme von Formalinfixierter Hefe, durch 2-stündiges Kultivieren von MD-APCs zur Selektion adhärenter Zellen, Zugabe von Hefe in einem Verhältnis von 1/10 MD-APCs/Hefe und Inkubieren bei 37°C, 5%-CO₂-Atmosphäre während 2-3 Stunden, Fixieren durch die May-Grünwald-Giemsa (MGG)-Färhung, und quantitative Bestimmung des Prozentsatzes phagozytischer MD-APCs beispielsweise durch mikroskopische Analyse,
- sie haben die Eigenschaft, die Proliferation allogener Lymphozyten zu stimulieren, wie durch den folgenden Test bestimmt:
allogene primäre Lymphozyten-Mischreaktion (MLR) wurde in Mikrotiterplatten mit 96 Vertiefungen durchgeführt, indem verschiedene Anzahl (2 × 10³ bis 2 × 10⁵ in 100 µl Medium/Vertiefung) MD-APCs zu 2 × 10⁵ allogenen T-Zellen, die vom Buffy coat gereinigt wurden, in 100 µl Medium/Vertiefung zugegeben wurden und nach 5-tägiger Inkubation bei 37°C die Zellproliferation nach einem kolorimetrischen Verfahren, wie die Spaltung von Tetrazoliumsalz WST-1 (leicht rot) zu Formozan (dunkelrot), oder wie Brdu-Einbau während der DNA-Synthese, bestimmt wurde.

## Revendications

1. Procédé pour préparer une composition comprenant des cellules présentatrices d'antigènes dérivées de monocytes (MDAPCs), ledit procédé comprenant les étapes consistant à :
- partir d'une composition contenant des leucocytes dérivés de donneurs sains
ou de patients et obtenus à partir de sang périphérique par aphérèse
- retirer les plaquettes et anticoagulant du produit de l'aphérése, tel que par centrifugation des produits de l'aphérèse
- isoler les cellules mononucléaires (monocytes + lymphocytes) des globules rouges et granulocytes de manière à avoir moins de 10 % de granulocytes et moins de 5 % de globules rouges,
- cultiver les cellules mononucléaires obtenues à l'étape précédente en les plaçant dans des sacs hydrophobes dans un milieu de culture approprié contenant, en tant que seuls ligands chimiques ayant des récepteurs sur la membrane des cellules mononucléaires :
- de l'histamine ou un agoniste du récepteur de l'histamine (II1 en action) et un antagoniste de II2
- ou de l'IL-13
en combinaison avec du GM-CSF exogène supplémentaire,
pendant une durée suffisante pour obtenir des MDAPCs différenciées, de préférence pendant environ 5 à 15 jours, et éventuellement séparer les MDAPCs des lymphocytes, et récupérer les MDAPCs et les lymphocytes.

2. Procédé selon la revendication 1, dans lequel le retrait des plaquettes et anticoagulant du produit de l'aphérèse est tel qu'obtenu par centrifugation des produits de l'aphérèse pendant 10 minutes à 280 g.

3. Procédé selon la revendication 1 ou 2, dans lequel le milieu de culture contient
- de l'histamine ou un agoniste du récepteur à l'histamine (III en action)
- un antagoniste 112
en combinaison avec du GM-CSF exogène, supplémentaire.

4. Procédé selon la revendication 1 ou 2, dans lequel le milieu de culture contient
- de l'IL-13
en combinaison avec du GM-CSF exogène, supplémentaire.

5. Procédé selon l'une quelconque des revendications 1 à 3, dans lequel le milieu de culture contient de l'histamine et un antagoniste H₂, en combinaison avec du GM-CSF supplémentaire, l'histamine étant présente à une concentration d'environ 10⁻² M à environ 10⁻⁶ M, et de préférence d'environ 10⁻⁴ M, l'antagoniste H₂ étant présent à une concentration d'environ 10⁻⁴ M à environ 10⁻⁹ M, et de préférence d'environ 10⁻⁶ M, et le GM-CSF supplémentaire étant présent à une concentration d'environ 50 U/ml à environ 1000 U/ml, de préférence d'environ 500 U/ml.

6. Procédé selon l'une quelconque des revendication 1 à 3 ou 5, dans lequel :
- l'agoniste du récepteur à l'histamine (H₁ en action) est la 2 méthyl-histamine, et / ou
- l'antagoniste H₂ est choisi parmi le groupe constitué de : la cimétidine, la tiotidine, le burinamide, le métiamide et la ranitidine.

7. Procédé selon l'une quelconque des revendications 1, 2 ou 4, dans lequel le milieu de culture contient de l'IL-13 et du GM-CSF exogène supplémentaire, ledit GM-CSF supplémentaire étant présent à une concentration d'environ 50 U/ml à environ 1000 U/ml, et de préférence d'environ 500 U/ml.

8. Procédé selon l'une quelconque des revendications 1 à 7, dans lequel on ajoute au milieu de culture des MDAPCs au moins un des éléments suivants :
- des antigènes bruts, par exemple de la membrane de tumeur autologue, des cellules tumorales tuées, des capsides bactériennes, des broyats viraux débarrassés d'acides nucléiques,
- des peptides spécifiques contre lesquels une réponse immunitaire est souhaitée,
- de l'ADNc ou un matériel génétique lié à des vecteurs (par exemple de la polylysine gluconatée) pour permettre la transfection des MDAPCs avec un matériel codant pour le peptide ou la protéine pertinent(c) devant être présenté(e) sur la membrane des MDAPCs et contre lequel (laquelle) une réponse immunitaire est souhaitée,
- des anticorps bispécifiques ciblant, d'une part un antigène de surface des MDAPCs, et d'autre part un antigène pertinent contre lequel une réponse immunitaire est souhaitée.

9. Cellules présentatrices d'antigènes dérivées de monocytes (MDAPCs) obtenues selon un procédé de l'une quelconque des revendications 1 à 8.

10. MDAPCs selon la revendication 9 qui possèdent les propriétés suivantes :
- elles présentent sur leur surface :
• les antigènes CD14 et CD64 avec une intensité moyenne d'environ 20 à environ 200,
• les antigènes CD80 et CD86 avec une intensité moyenne d'environ 20 à environ 200,
• l'antigène CD40 et le récepteur du mannose avec une intensité moyenne d'environ 50 à environ 500,
• les antigènes CD 1 a et CD 1 c avec une intensité moyenne inférieure à 20, la présence et les intensités moyennes respectivement de CD14, CD64, CD80, CD86, CD40, du récepteur du mannose, de CD1a et CD1c étant déterminées par coloration d'immunofluorescence et analyse par cytométrie en flux,
- elles présentent une propriété de phagocytose telle que déterminée par le test suivant:
ladite capacité de phagocytose étant évaluée par une capture de levures fixées à la formaline en cultivant des macrophages pendant 2 heures, en ajoutant les levures dans un rapport macrophages / levures de 1/10 et en incubant à 37°C dans une atmosphère de 5 % de CO₂ pendant 2-3 heures, en fixant par la coloration de May-Grünwald-Giemsa (MGG), et le pourcentage de MDAPCs phagocytaires étant quantifié par exemple par analyse microscopique,
- elles possèdent la propriété de stimuler la prolifération de lymphocytes allogéniques telle que déterminée par le test suivant :
une réaction lymphocytaire mixte (RLM) allogénique primaire est effectuée dans des plaques de micro-titration à 96 puits en ajoutant différents nombres (2 × 10³ à 2 × 10⁵ dans 100 µl de milieu / puits) de MDAPCs à une quantité de 2 × 10⁵ dans 100 µl de milieu / puits de lymphocytes T allogéniques purifiés à partir de concentré leucocyto-plaquettaire et après 5 jours d'incubation à 37°C, la prolifération cellulaire est estimée par un procédé colorimétrique, tel que l'hydrolyse du sel de tétrazolium WST-1 (Bochringer Mannheim, Allemagne), (légèrement rouge) en Formozan (rouge sombre).

11. MDAPCs selon la revendication 9 ou 10, présentant une capacité de phagocytose de 80 %.

12. MDAPCs selon l'une quelconque des revendications 9 à 11, qui présentent, sur leur surface, l'antigène MHC-II avec une intensité moyenne d'environ 100 à environ 400, telle que déterminée par coloration d'immunofluorescenee et analyse par cytométrie en flux.

13. MDAPCs selon l'une quelconque des revendications 9 à 12, qui sont essentiellement dépourvues de l'antigène de surface CD83, tel que déterminé par coloration d'immunofluorescence et analyse par cytométrie en flux.

14. MDAPCs selon l'une quelconque des revendications 9 à 13, qui présentent des propriétés adhérentes telles que déterminées par le test suivant :
les macrophages sont cultivés pendant 2 h dans du milieu de culture (IMDM
ou RPMI) sur des flacons en plastique et le pourcentage (%) de cellules adhérentes est quantifié par exemple par analyse microscopique.

15. Cellules présentatrices d'antigènes dérivées de monocytes (MDAPCs) qui possèdent les propriétés suivantes :
- elles présentent sur leur surface :
• les antigènes CD14 et CD64 avec une intensité moyenne d'environ 20 à environ 200,
• les antigènes CD80 et CD86 avec une intensité moyenne d'environ 20 à environ 200,
• l'antigène CD40 et le récepteur du mannose avec une intensité moyenne de 50 à 500,
• les antigènes CD1a et CD1c avec une intensité moyenne inférieure à 20, la présence et les intensités moyennes respectivement de CD14, CD64, CD80, CD86, CD40, du récepteur du mannose, de CD1a et CD1c étant déterminées par coloration d'immunofluorescence et analyse par cytométrie en flux,
- elles présentent une propriété de phagocytose telle que déterminée par le test suivant :
ladite capacité de phagocytose étant évaluée par une capture de levures fixées à la formaline en cultivant des macrophages pendant 2 heures, en ajoutant les levures dans un rapport macrophages / levures de 1/10 et en incubant à 37°C dans une atmosphère de 5 % de CO₂ pendant 2-3 heures, en fixant par la coloration de May-Grünwald-Giemsa (MGG), et le pourcentage de MDAPCs phagocytaires étant quantifié par exemple par analyse microscopique,
- elles possèdent la propriété de stimuler la prolifération de lymphocytes allogéniques telle que déterminée par le test suivant:
une réaction lymphocytaire mixte (RLM) allogénique primaire est effectuée dans des plaques de micro-titration à 96 puits en ajoutant différents nombres (2 × 10³ à 2 × 10⁵ dans 100 µl de milieu / puits) de MDAPCs à une quantité de 2 × 10⁵ dans 100 µl de milieu / puits de lymphocytes T allogéniques purifiés à partir de concentré leucocyto-plaquettaire et après 5 jours d'incubation à 37°C, la prolifération cellulaire est estimée par un procédé colorimétrique, tel que l'hydrolyse du sel de tétrazolium WST-1 (Boehringer Mannheim, Allemagne), (légèrement rouge) en Formozan (rouge sombre).

16. MDAPCs selon la revendication 15, présentant une capacité de phagocytose de 80 %.

17. MDAPCs selon la revendication 15 ou 16, qui présentent, sur leur surface, l'antigène MHC-II avec une intensité moyenne d'environ 100 à environ 400, telle que déterminée par coloration d'immunofluorescence et analyse par cytométrie en flux.

18. MDAPCs selon l'une quelconque des revendications 15 à 17, qui sont essentiellement dépourvues de l'antigène de surface CD83, tel que déterminé par coloration d'immunofluorescence et analyse par cytométrie en flux.

19. MDAPCs selon l'une quelconque des revendications 15 à 18, qui présentent des propriétés adhérentes telles que déterminées par le test suivant :
les macrophages sont cultivés pendant 2 h dans du milieu de culture (IMDM ou RPMI) sur des flacons en plastique et le pourcentage (%) de cellules adhérentes est quantifié par exemple par analyse microscopique.

20. Compositions pharmaceutiques contenant des MDAPCs selon l'une quelconque des revendications 9 à 19 en tant que substance active.

21. Compositions de vaccin cellulaire contenant des MDAPCs selon l'une quelconque des revendications 9 à 19 en tant que substance active.

22. Milieu contenant les éléments nécessaires pour la croissance et la différentiation de monocytes en MDAPCs selon un procédé de l'une quelconque des revendications 1 à 8, contenant en tant que seuls ligands chimiques ayant des récepteurs sur la membrane des cellules mononucléaires :
- de l'histamine ou un agoniste du récepteur de l'histamine (H₁ en action), et un antagoniste H₂ tel que la cimétidine,
- ou de l'IL-13
en combinaison avec du GM-CSF exogène, supplémentaire.

23. Milieu selon la revendication 21, contenant :
- de l'histamine ou un agoniste du récepteur de l'histamine (H₁ en action), et
- un antagoniste H₂, et
du GM-CSF exogène, supplémentaire.

24. Milieu selon l'une quelconque des revendications 21 à 23, contenant de l'histamine et un antagoniste H₂, en combinaison avec du GM-CSF exogène supplémentaire, l'histamine étant présente à une concentration d'environ 10⁻² M à environ 10⁻⁶ M, et de préférence d'environ 10⁻⁴ M, l'antagoniste H₂ étant présent à une concentration d'environ 10⁻⁴ M à environ 10⁹ M, et de préférence d'environ 10⁻⁶ M, et le GM-CSF exogène supplémentaire étant présent à une concentration d'environ 50 U/ml à environ 1000 U/ml, de préférence d'environ 500 U/ml.

25. Milieu selon l'une quelconque des revendication 21 à 24, dans lequel :
- l'agoniste du récepteur de l'histamine (H₁ en action) est la 2 méthyl-histamine, et / ou
- l'antagoniste II₂ est choisi parmi le groupe constitué de : la cimétidine, la tiotidine, le burinamide, le métiamide et la ranitidine.

26. Milieu selon la revendication 25, dans lequel l'antagoniste H₂ est la cimétidine.

27. Milieu selon la revendication 22, dans lequel on ajoute de l'IL-13 et du GM-CSF au milieu de culture, le GM-CSF exogène supplémentaire étant présent à une concentration d'environ 50 U/ml à environ 1000 U/ml, et de préférence d'environ 500 U/ml.

28. Processeur cellulaire ou kit contenant :
- des moyens pour la récupération de lymphocytes et de monocytes sans contaminants,
- un tampon approprié et des solutions de lavage et éventuellement des moyens appropriés pour la conservation des cellules présentatrices d'antigènes dérivées de monocytes (MDAPCs),
- de moyens pour préparer une culture pour les monocytes et éventuellement les lymphocytes et contenant en tant que seuls ligands chimiques ayant des récepteurs sur la membrane des cellules mononucléaires
- de l'histamine ou un agoniste du récepteur de l'histamine (H₁ en action), et un antagoniste H₂ tel que la cimétidine,
- ou de l'IL-13,
en combinaison avec du GM-CSF,
- éventuellement des moyens pour la transfection des cellules cultivées et des moyens pour cibler les antigènes vers les MDAPCs.

29. Processeur cellulaire ou kit selon la revendication 28, contenant :
- de l'histamine ou un agoniste du récepteur de l'histamine (H₁ en action), et
- un antagoniste H₂,
en combinaison avec du GM-CSF.

30. Processeur cellulaire ou kit selon la revendication 28, contenant de l'II.-13 et du GM-CSF.

31. Processeur cellulaire ou kit selon l'une quelconque des revendications 26 à 28, contenant des moyens pour préparer une culture pour les monocytes et éventuellement les lymphocytes, et contenant de l'histamine et de la cimétidine.

32. Processeur cellulaire ou kit selon la revendication 30, contenant des moyens pour préparer une culture pour les monocytes et éventuellement les lymphocytes, et contenant de I'IL-13 et du GM-CSF.

33. Processeur cellulaire ou kit selon l'une quelconque des revendications 28 à 32, contenant :
- des moyens pour récupérer et centrifuger le sang pour obtenir un concentrat de leucocytes,
- des moyens pour séparer les lymphocytes et monocytes des autres globules blancs et pour éliminer les globules rouges contaminants,
- un milieu de culture pour les MDAPCs et éventuellement les lymphocytes avec des compléments et en particulier, en tant que seuls ligands chimiques ayant des récepteurs sur la membrane des cellules mononucléaires de l'histamine ou un agoniste du récepteur de l'histamine (H₁ en action), et un antagoniste H₂,
en combinaison avec du GM-CSF,
- des moyens appropriés pour la conservation des MDAPCs,
- un tampon approprié et une solution de lavage.

34. Processeur cellulaire ou kit selon la revendication 28, contenant un milieu de culture pour les MDAPCs et éventuellement les lymphocytes, avec les compléments étant l'histamine et la cimétidine en combinaison avec du GM-CSF.

35. Processeur cellulaire ou kit selon la revendication 27, contenant un milieu de culture pour les MDAPCs et éventuellement les lymphocytes, avec les compléments étant l'1L-13 et le GM-CSF.

36. Produits contenant des MDAPCs selon l'une quelconque des revendications 9 à 17, et des lymphocytes, en tant que préparation combinée pour une utilisation simultanée, séparée ou séquentielle en thérapie cellulaire.

37. Produits selon la revendication 36, **caractérisés en ce qu'**ils contiennent les MDAPCs et les lymphocytes dans un rapport d'au moins 20 % à 50 % de MDAPCs exprimé en nombre de cellules.

38. Utilisation de MDAPCs selon l'une quelconque des revendications 9 à 17, pour la préparation d'un médicament pour le traitement du cancer et des infections par des pathogènes.

39. Utilisation de MDAPCs selon l'une quelconque des revendications 9 à 17, pour la préparation d'un médicament pour le traitement d'une maladie impliquant la phagocytose, et la stimulation de la prolifération des lymphocytes T.

40. Utilisation selon la revendication 38 ou 39, **caractérisée en ce que** ledit médicament contient d'environ 10⁸ à environ 5 × 10⁹ MDAPCs.

41. Utilisation selon l'une quelconque des revendications 38 à 40, **caractérisée en ce que** ledit médicament contient également des lymphocytes.

42. Utilisation de l'histamine ou d'un agoniste du récepteur de l'histamine (H₁ en action), et d'un antagoniste H₂, en particulier la cimétidine, ou d'IL-13, en combinaison avec GM-CSF, pour la préparation in vitro de cellules présentatrices d'antigènes dérivées de monocytes (MDAPCs) possédant les propriétés suivantes :
- elles présentent sur leur surface :
les antigènes CD14 et CD64 avec une intensité moyenne d'environ 20 à environ 200,
les antigènes CD80 et CD86 avec une intensité moyenne d'environ 20 à environ 200,
l'antigène CD40 et le récepteur du mannose avec une intensité moyenne de 50 à 500,
les antigènes CD1a et CD1c avec une intensité moyenne supérieure à 20, la présence et les intensités moyennes respectivement de CD14, CD64, CD80, CD86, CD40, du récepteur du mannose, de CD1a et CD1c étant déterminées par coloration d'immunofluorescence et analyse par cytométrie en flux,
- elles présentent une propriété de phagocytose élevée telle que déterminée par le test suivant :
ladite capacité de phagocytose étant évaluée par une capture de levures fixées à la formaline,
en cultivant des MDAPCs pendant 2 heures pour sélectionner les cellules adhérentes, en ajoutant les levures dans un rapport MDAPCsm / levures de 1/10 et en incubant à 37°C dans une atmosphère de 5 % de CO₂ pendant 2-3 heures, en fixant par la coloration de May-Grünwald-Giemsa (MGG), et le pourcentage de MDAPCs phagocytaires étant quantifié par exemple par analyse microscopique,
- elles possèdent la propriété de stimuler la prolifération de lymphocytes allogéniques telle que déterminée par le test suivant :
une réaction lymphocytaire mixte (RLM) allogénique primaire est effectuée dans des plaques de micro-titration à 96 puits en ajoutant différents nombres (2 × 10³ à 2 × 10⁵ dans 100 µl de milieu / puits) de MDAPCs à une quantité de 2 × 10⁵ dans 100 µl de milieu / puits de lymphocytes T allogéniques purifiés à partir de concentré leucocyto-plaquettaire et après 5 jours d'incubation à 37°C, la prolifération cellulaire est estimée par un procédé colorimétrique, tel que le clivage du sel de tétrazolium WST-1, (légèrement rouge) en Formozan (rouge sombre) ou tel que l'incorporation de Brdu lors de la synthèse d'ADN.
